# EUROPEAN PATENT SPECIFICATION

(11) **EP 3 140 027 B1**
(45) Date of publication and mention of the grant of the patent: **26.12.2018**
(21) Application number: 15732347.8
(22) Date of filing: 08.05.2015
(51) Int. Cl.: B01D 53/32, C07C 273/00

(54) **SYSTEM AND METHOD FOR CLEANING A GAS STREAM FROM A UREA PLANT SOLIDIFICATION UNIT**
SYSTEM UND VERFAHREN ZUR REINIGUNG EINES GASSTROMS AUS EINE HARNSTOFFVERFESTIGUNGSANLAGE
SYSTEME ET PROCEDE POUR L'EPURATION D'UN FLUX DE GAZ PROVENANT D'UNE UNITE DE SOLIDIFICATION D'INSTALLATION DE PRODUCTION D'UREE

(30) Priority: 09.05.2014 IT MI20140849
(43) Date of publication of application: 15.03.2017
(73) Proprietor: Saipem S.p.A., 20097 San Donato Milanese (Milano) (IT)
(72) Inventor: BRUNO, Lorenzo, 20097 San Donato Milanese (IT); CARLESSI, Lino, 24044 Dalmine (IT)
(74) Representative: Cernuzzi, Daniele
(86) International application number: PCT/IB2015/053385
(87) International publication number: WO 2015/170293

(56) References cited:
- EP-A1- 2 535 115
- EP-A2- 0 640 377
- WO-A1-2007/048998
- WO-A1-2009/065534
- WO-A1-2011/099844
- WO-A1-2011/149914
- JP-A- S5 169 478
- US-A1- 2006 226 373
- US-A1- 2013 287 669

## Description

### TECHNICAL FIELD

The present invention relates to a system and method for cleaning a gas stream from a solidification unit of a urea plant.

### BACKGROUND ART

In a typical industrial urea production plant (urea plant), the urea is produced in a synthesis reactor, then progressively concentrated (up to values of approximately 96-99.7%), and finally solidified in a solidification section, which normally consists of a granulator or prilling tower, from which a final commercial product is obtained in the form of urea granules or prills.

Normally, the urea sent to the granulator or prilling tower is in a liquid state and solidifies by means of a stream of ambient air.

When the air used for solidification leaves the solidification section, it contains urea powder and ammonia released by the liquid urea during the cooling and solidification process.

Before releasing this air into the atmosphere, it is therefore opportune to purify it by removing the urea powder and ammonia it contains.

To this end, the use of two scrubbers working in series on the gas stream to be purified is both known and common.

In a first scrubber, used for removing urea powder, an aqueous solution containing liquid urea is recirculated; a urea solution is drawn off from the bottom of this first scrubber, of which a part is recirculated to the top of the scrubber and a part is sent to the urea plant to be recovered.

The gas stream (purified of urea powder) leaving the first scrubber is sent to a second scrubber where ammonia is removed by a recirculating solution containing, for example, ammonium sulphate and ammonium bisulphate, and where fresh sulphuric acid is periodically added to maintain the correct ratio between ammonium sulphate and ammonium bisulphate.

The gas stream leaving the second scrubber is essentially air purified of both urea powder and ammonia, and which can therefore be released into the atmosphere via a stack.

To overcome head loss, a fan is located downstream of the second scrubber.

The system just mentioned, and similar ones for that matter, are not without drawbacks, basically related to the use of scrubbers.

First of all, the recovery of urea from the solution of liquid urea achieved by the first scrubber entails a significant expenditure of energy, and consequently of costs, given the high level of water content.

Furthermore, the scrubbers generally have low removal efficiency, with consequent fouling and/or corrosion problems on the fans located downstream of the scrubbers and the need for relatively frequent periodic washing to eliminate encrustations and deposits.

Due to their moderate efficiency, the scrubbers call for a significant consumption of makeup water, necessary for cleaning the demisters commonly used in the scrubbers to aid the separation of the drops of liquid entrained in the gas current.

Lastly, especially in latest-generation urea plants, which produce high flows of air to be purified (even exceeding 800,000 Nm3/h), distribution of the gas stream in the scrubbers is poor, with a consequent drop in efficiency; in particular, there is low efficiency in the scrubber for removing gaseous ammonia due to the formation of aerosols that escape from the common demisters with coalescers; furthermore, aerosols are strongly pH dependent and their formation is difficult to control.

WO2011099844A1 discloses a system and a method for the removal of ammonia and urea dust from the off-gas of a finishing section of a urea production plant. The off-gas is contacted with a solid adsorbent capable of physically adsorbing ammonia, and then the adsorbent having ammonia adsorbed thereon and the urea dust are separated from the gas stream in a solid-gas separation step, by means of baghouse filters or cyclones.

### DISCLOSURE OF INVENTION

One object of the present invention is therefore to provide a system and method for cleaning a gas stream from a solidification unit of a urea plant, in particular for removing urea powder and gaseous ammonia from said gas stream, without the described drawbacks of the known art.

In particular, an object of the invention is to provide a cleaning system and method that, with respect to known solutions, has high efficiency, reduced head loss and energy consumption, and therefore lower costs, and does not require components replenishment.

The present invention therefore relates to a urea plant comprising a solidification unit and a cleaning system for cleaning a gas stream from said solidification unit and containing urea powder and ammonia, as claimed in claim 1; and to a method for cleaning a gas stream from a solidification unit of a urea plant and containing urea powder and ammonia, as claimed in claim 10.

Additional preferred features of the invention are indicated in the dependent claims.

In brief, in accordance with the invention, both the scrubbers usually used for purifying the cooling air leaving the solidification unit are replaced with as many electrostatic precipitators (filters) and precisely: a 'dry' electrostatic precipitator to recover the urea powder, and a 'wet' electrostatic precipitator for removing gaseous ammonia.

The electrostatic precipitators used in accordance with the invention have high removal efficiency, high flow speeds for the gas stream and very low head loss.

In addition, the electrostatic precipitators work by using substances that are already present and do not need others to be added.

The high efficiency of the electrostatic precipitators enables reducing problems of environmental emission of urea powder and gaseous ammonia and limiting the fouling and corrosion of the fans used for the circulation of the gas stream.

The efficiency of the electrostatic precipitators can also be improved by conditioning the gas stream that is fed to the electrostatic precipitators.

In particular, in the 'dry' electrostatic precipitator for removing urea powder, conditioning is performed using ammonia vapours (ammonia gas conditioning), already present in the gas stream to be purified or, if necessary, introduced from the outside, preferably via atomization; and/or using other substances also present in the cleaning system, e.g. ammonium bisulphate.

In the 'wet' electrostatic precipitator for removing gaseous ammonia, a gas, for example ammonium bisulphate gas, which is subsequently recovered as ammonium sulphate, is atomized for the conditioning.

The flow speed of the stream passing through the electrostatic precipitators enables having compact apparatuses with similar or smaller sizes than the scrubbers, while the low head losses enable using less powerful and therefore less expensive fans located downstream of the electrostatic precipitators.

In short, the main advantages of the invention are the following:
- lesser head losses due to greater efficiency;
- lower costs and less energy expended for eliminating water;
- lesser fouling and clogging problems with fans and demisters.

### BRIEF DESCRIPTION OF THE DRAWINGS

Further characteristics and advantages of the present invention will become clear from the description of the following non-limitative embodiments, with reference to the figures in the accompanying drawings, in which:
- Figure 1 is a schematic view of a first embodiment of a system for cleaning a gas stream from a solidification unit of a urea plant, in accordance with the invention; and
- Figure 2 is a schematic view of a second embodiment of the cleaning system in accordance with the invention.

### BEST MODE FOR CARRYING OUT THE INVENTION

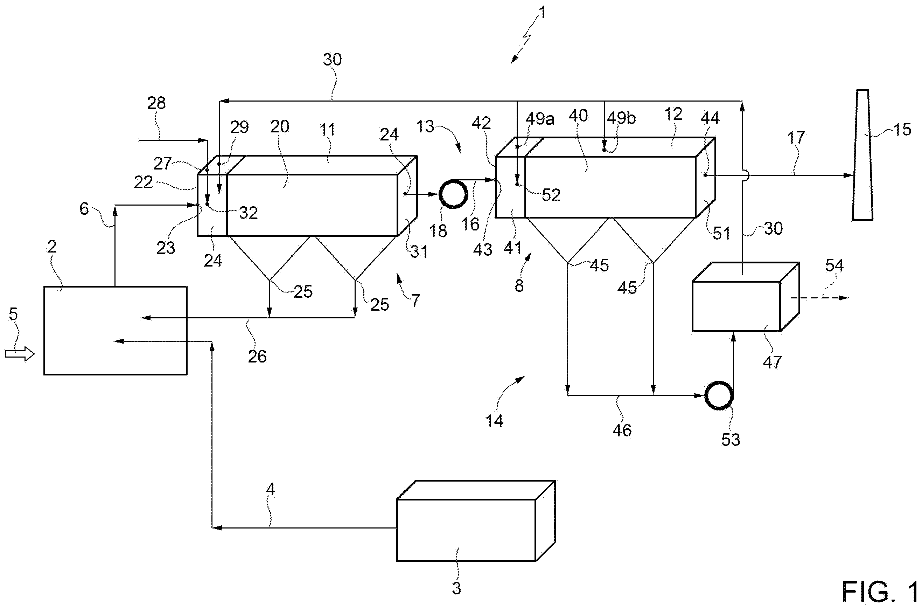

Figure 1 schematically shows a cleaning system 1 for processing a gas stream leaving a solidification unit 2 of a urea plant 3, in particular for removing urea powder and gaseous ammonia from said gas stream.

The urea plant 3 is, in itself, essentially known and shall therefore neither be described nor illustrated in detail. In general terms, the urea plant 3 comprises: a urea synthesis reactor where the reaction of urea synthesis from ammonia and carbon dioxide takes place; recovery sections (in particular, a high-pressure section, a medium-pressure section and a low-pressure section), in which the urea solution produced in the synthesis reactor is progressively concentrated, with the removal of water and unreacted ammonia and carbon dioxide, and recirculation of the recovered components; and a vacuum section equipped with a vacuum system and connected to a waste-water treatment section.

The urea plant 3 is connected via a urea feed line 4 to the solidification unit 2, which includes, for example, a granulator or a prilling tower to which the molten urea produced in the urea plant 3 is sent to obtain solid urea in granules or prills.

The solidification unit 2 is fed with a stream of cooling air, schematically represented by the arrow 5 in Figure 1, which enters the solidification unit 2 through specially provided air inlet openings, for the purpose of solidifying the molten urea.

From the solidification unit 2 a gas stream exits that is basically formed by the cooling air used in the solidification unit 2 and contains urea powder and gaseous ammonia released by the urea during the cooling and solidification process.

The gas stream leaving the solidification unit 2 is sent to the cleaning system 1 through a gas line 6 that operationally connects the solidification unit 2 to the cleaning system 1.

The cleaning system 1 comprises a urea powder removal device 7 and an ammonia removal device 8, to respectively remove urea powder and ammonia from a gas stream leaving the solidification unit 2.

The two removal devices 7, 8 are designed to treat respective contaminants, i.e. urea powder or ammonia, and to mainly remove the respective contaminants (urea powder or ammonia).

More precisely, the cleaning system 1 comprises a single first removal device 7 dedicated to the removal of urea powder and a single second removal device 8 dedicated to the removal of ammonia.

In accordance with the invention, the removal devices 7, 8 consist of respective electrostatic precipitators 11, 12, designed to substantially remove all or at least most of the contaminant treated in the removal device 7, 8, i.e. urea powder and ammonia.

As is known, an electrostatic precipitator (or electrostatic filter) is a purification apparatus that implements a removal process in which (solid or liquid) pollutant particles are removed from a gas stream by application of an induced electric field.

In general, an electrostatic precipitator basically comprises:
- discharge and emission electrodes, generally of filiform shape;
- collector electrodes, usually in the form of plates of various shapes (for example, flat or tubular plates), arranged parallel to the discharge electrodes;
- a power supply unit, for generating the voltage levels required by the purification process;
- an apparatus for periodic cleaning of the collector electrodes; and
- structures for housing the electrodes, distribution of the gas streams in input and output, and collection and/or discharge of the particles removed.

An electrostatic precipitator operates by means of a potential difference induced between the discharge electrodes and the collector electrodes so as to generate a strong electric field close to the discharge electrodes and around them, which causes ionization of the gas stream containing the contaminating particles. The ions produced collide with the contaminant particles in suspension and give them an electric charge; the charged particles are then attracted towards the collector electrodes were they are captured and then removed, for example by shaking the collector electrodes or using washing solutions that lap the collector electrodes.

An electrostatic precipitator may comprise a number of sections or fields (i.e. groups of discharge electrodes and collector electrodes) arranged in series to treat the gas stream in succession.

In greater detail, the cleaning system 1 comprises: a first electrostatic precipitator 11 and a second electrostatic precipitator 12 to respectively remove urea powder and ammonia from the gas stream leaving the solidification unit 2; an air treatment circuit 13; and a recirculation circuit 14.

Since two electrostatic precipitators 11, 12 are used, other apparatuses, in particular scrubbers, dedicated to the removal of urea powder or ammonia from the gas stream leaving the solidification unit 2 are not included in the cleaning system 1.

The two electrostatic precipitators 11, 12 are arranged in series along the gas treatment circuit 13 that connects the solidification unit 2 to a stack 15, which discharges into the atmosphere.

In particular, the gas treatment circuit 13 comprises: a gas line 6, which connects the solidification unit 2 to the first electrostatic precipitator 11; a connecting line 16, which connects the two electrostatic precipitators 11, 12; if necessary, a discharge line 17 connected to the stack 15; and at least one fan 18, preferably located along the connecting line 16 between the electrostatic precipitators 11, 12.

In the preferred embodiment shown in Figure 1, electrostatic precipitator 11 is located upstream of electrostatic precipitator 12 along the gas treatment circuit 13.

The function of electrostatic precipitator 11 is to remove urea powder from the gas stream leaving the solidification unit 2; the removal of urea powder from the gas stream in electrostatic precipitator 11 takes place due to an electrostatic effect and is possibly aided by conditioning of the gas stream.

The electrostatic precipitator 11 for removing urea powder is a 'dry' electrostatic precipitator.

Here and in the following, a 'dry' electrostatic precipitator means an electrostatic precipitator operating so as to recover a substantially solid product (even if not entirely dry) from the gas stream.

Electrostatic precipitator 11 comprises an electrode region 20, which houses discharge electrodes and collector electrodes and where the removal process takes place due to the effect of the electric field induced between the electrodes, and a conditioning region 21, connected to an inlet end 22 of electrostatic precipitator 11 and upstream of the electrode region 20 along a path of the gas stream in electrostatic precipitator 11 and where the gas stream is optionally supplemented with a conditioner that improves the efficiency of the removal process before passing through the electrode region 20.

Electrostatic precipitator 11 has: a gas inlet 23 connected to the gas line 6; a gas outlet 24 connected to the connecting line 16; one or more bottom outlets 25 connected by a return line 26 to the solidification unit 2; an auxiliary inlet 27 connected to a conditioning line 28; and a recirculation inlet 29 connected to a recirculation line 30.

Advantageously, the gas inlet 23, the auxiliary inlet 27 and the recirculation inlet 29 are located at the inlet end 22 of the conditioning region 21, while the gas outlet 24 is located at an outlet end 31 of the electrostatic precipitator 11, and the bottom outlets 25 are located beneath the electrode region 20 (for example, beneath respective fields or sections of electrostatic precipitator 11).

Electrostatic precipitator 11 has high efficiency in removing urea powder also due to the presence of ammonia vapours in the gas stream coming from the solidification unit 2 (granulator or prilling tower) and which condition the gas stream.

However, it is possible to add further conditioner to increase the yield of the removal process, in particular more ammonia (ammonia solution or ammonia vapours) or ammonium bisulphate (or even other substances).

For example, conditioner could be introduced from the outside, through the conditioning line 28 and an injector device 32, located at the auxiliary inlet 27 and advantageously provided with an atomizer.

In the case where the solidification unit 2 is a granulator, the possible addition of an ammonia solution (or ammonia vapours) for conditioning could be already included in the solidification unit 2 to improve the subsequent distribution.

The levels of ammonia necessary for good removal performance are around 50-60 ppm, levels that are in fact already present in the gas stream without the need for further action.

With conditioning, the flow speeds are around 2-4 m/s with removal levels of 10 mg/Nm3.

Alternatively, the conditioner, for example ammonium bisulphate, could be injected into the conditioning region 21 from the recirculation inlet 29, connected by a recirculation line 30 to a recovery unit 47, described in greater detail hereinafter.

However, in addition to the possible use of a conditioner, the efficiency of an electrostatic precipitator also generally depends on the number of fields or sections (i.e. groups of discharge electrodes and collector electrodes arranged in series) through which the gas stream passes.

Electrostatic precipitator 11 preferably has at least 2 or 3 fields.

The removal efficiency of electrostatic precipitator 11 is already acceptable at values of approximately 95-98% if the cleaning system 1 comprises a successive ammonia removal unit (electrostatic precipitator 12) that also recovers the last traces of urea powder.

The 'dry' electrostatic precipitator 11 is preferably (but not necessarily) a flat-plate electrostatic precipitator.

The function of electrostatic precipitator 12 is to remove ammonia from the gas stream leaving the solidification unit 2; the removal of ammonia in electrostatic precipitator 12 takes place due to an electrostatic effect and is possibly aided by conditioning of the gas stream.

The electrostatic precipitator for removing ammonia is a 'wet' electrostatic precipitator.

Here and in the following, a 'wet' electrostatic precipitator means an electrostatic precipitator operating so as to recover a liquid (e.g. a solution) or partially liquid (e.g. sludge) product from the gas stream.

Electrostatic precipitator 12 also comprises a electrode region 40, which houses discharge electrodes and collector electrodes and where the removal process takes place due to the effect of the electric field induced between the electrodes, and a conditioning region 41, located at an inlet end 42 of the electrostatic precipitator 12 and upstream of the electrode region 40 along a path of the gas stream in electrostatic precipitator 12 and where the gas stream is supplemented with a conditioner that improves the efficiency of the removal process before passing through the electrode region 40.

Electrostatic precipitator 12 has: a gas inlet 43 connected to the connecting line 16; a gas outlet 44 connected to the discharge line 17 and therefore to the stack 15; one or more bottom outlets 45 connected by a recovery line 46 to a recovery unit 47; and one or more recirculation inlets 49a and 49b connected to the recirculation line 30.

Gas inlet 43 and a first recirculation inlet 49a are located at the inlet end 42 in the conditioning region 41, a second recirculation inlet 49b is located in the electrode region 40, gas outlet 44 is located at an outlet end 51 of the electrostatic precipitator 12, and the bottom outlets 45 are located beneath the electrode region 40 (for example, beneath respective fields or sections of electrostatic precipitator 12).

The ammonia removal efficiency of electrostatic precipitator 12 is increased by conditioning with a conditioner, for example atomized ammonium bisulphate, fed to the inlet end 42 in the conditioning region 41 of electrostatic precipitator 12 via an injector device 52. It is understood however that other conditioners could be used.

Since, as happens when ammonium sulphate is used, the conditioner reacts with the ammonia during the ammonia removal process and forms a by-product containing the ammonium group, that by-product is recovered and processed to obtain the conditioner again, so as to be recirculated to the second electrostatic precipitator 12 and possibly to the first electrostatic precipitator 11 as well.

For example, whilst removing ammonia, ammonium bisulphate reacts with ammonia to give ammonium sulphate, which is recovered in solution from the bottom outlets 45.

Ammonium sulphate can be used as a by-product and therefore removed from the cleaning system 1, but in this case it is necessary to replenish sulphuric acid in the cleaning system 1 to reform ammonium bisulphate for use as a conditioner.

According to the invention, as shown in Figure 1, the ammonium sulphate is sent, via the recovery line 46 equipped with a circulation pump 53, to the recovery unit 47 where the ammonium sulphate is degraded (through heating) to form ammonium bisulphate and ammonia.

In the recovery unit 47, the solution of ammonium sulphate coming from electrostatic precipitator 12 is thermally treated (via opportune heating means) to produce ammonia in the gaseous phase, which can be sent to the urea plant 3 through a urea line 54 for the production of urea, and a solution of ammonium bisulphate (also containing a smaller quantity of ammonium sulphate), which is reused in electrostatic precipitator 12 as a conditioner for removing ammonia, as it is recirculated to electrostatic precipitator 12 through the recirculation line 30.

The recirculation line 30 is connected to both recirculation inlets 49a, 49b of electrostatic precipitator 12, and optionally (if conditioning with ammonium bisulphate is also contemplated in the first electrostatic precipitator 11) to recirculation inlet 29 of electrostatic precipitator 11 as well, through respective line portions.

For effective conditioning with the solution of ammonium bisulphate and effective ammonia removal, it is advantageous to atomize the solution of ammonium bisulphate at recirculation inlet 49a, ensuring adequate distribution, which enables reducing the contact time necessary to achieve high ammonia absorption efficiency.

Injector device 52, located on the recirculation line 30, for example at recirculation inlet 49a, is therefore equipped with an atomizer.

The conditioning enables having flow speeds in the order of 2-4 m/s, with consequent reductions in the size of electrostatic precipitator 12 and a final removal efficiency exceeding 99%.

To improve the distribution of ammonium bisulphate in the gas stream treated in the electrostatic precipitator 12, the ammonium bisulphate atomizer can be inserted along the connecting line 16 upstream of the fan 18; however, in this case, the amount of ammonium bisulphate injected must be limited to avoid the formation of liquids downstream of the fan 18.

There can be different types of electrostatic precipitator 12, even according to the flow rates of the gas stream to be treated; for example, a tubular, a circular-plate, or a flat-plate electrostatic precipitator.

Since (as shown in Figure 1) the electrostatic precipitators 11, 12 are used in series, it is advantageous to insert the fan 18 (or a number of fans 18) between the two electrostatic precipitators 11, 12, so as to avoid corrosion and the formation of condensation.

In use, a urea powder removal step and an ammonia removal step respectively take place in the two electrostatic precipitators 11, 12, these being entirely or mainly carried out in the respective electrostatic precipitator 11, 12, where substantially all or at least most of the contaminant treated in the removal step, i.e. urea powder and ammonia, is removed.

The solidification unit 2 receives molten urea from the urea plant 3 through the urea feed line 4 and is fed with cooling air 5. Solid urea (in granules or prills) is collected from the solidification unit 2 and a gas stream emitted that is basically formed by the cooling air used in the solidification unit 2 and containing urea powder and ammonia; the gas stream leaving the solidification unit 2 is sent to the cleaning system 1 and specifically to the first electrostatic precipitator 11 through the gas line 6.

The gas stream leaving the solidification unit 2 enters the first electrostatic precipitator 11 through gas inlet 23, is possibly conditioned by the injection of a conditioner (ammonia vapours and/or ammonium bisulphate solution), and passes through the electrode region 20 where the urea powder removal process takes place.

The application of a potential difference between the discharge electrodes and the collector electrodes of the electrode region 20 generates an electric field that causes ionization of the gas stream passing through the electrode region 20; the particles of urea present become charged and are attracted by the collector electrodes, after which they are discharged from the bottom outlets 25 and returned through the return line 26 to the solidification unit 2.

The gas stream exits from gas outlet 24, passes through the connecting line 16 (provided with the fan 18) and reaches the second electrostatic precipitator 12.

The gas stream enters the second electrostatic precipitator 12 through gas inlet 43, is conditioned by the injection of a conditioner (a solution of ammonium bisulphate), and passes through the electrode region 40 where the ammonia removal process takes place.

The application of a potential difference between the discharge electrodes and the collector electrodes of the electrode region 40 generates an electric field that causes ionization of the gas stream passing through the electrode region 40; the ammonia present becomes charged and is attracted by the collector electrodes, after which it is discharged from the bottom outlets 45.

If ammonium bisulphate is used as a conditioner, during the ammonia removal process, the ammonium bisulphate reacts with the ammonia to give ammonium sulphate; a solution of ammonium sulphate is thus recovered from the bottom outlets 45 and sent, through the recovery line 46, to the recovery unit 47. In the recovery unit 47, the solution of ammonium sulphate is thermally treated to degrade the ammonium sulphate and produce ammonia in the gaseous phase (to be recovered and returned to the urea plant 3 through the urea line 54) and ammonium bisulphate in solution, to be reused in the cleaning system 1 as a conditioner in the electrostatic precipitators 11, 12 (through the recirculation line 30).

A gas stream purified of urea powder and ammonia leaves the gas outlet 44 of the electrostatic precipitator 12 and is then discharged via the stack 15.

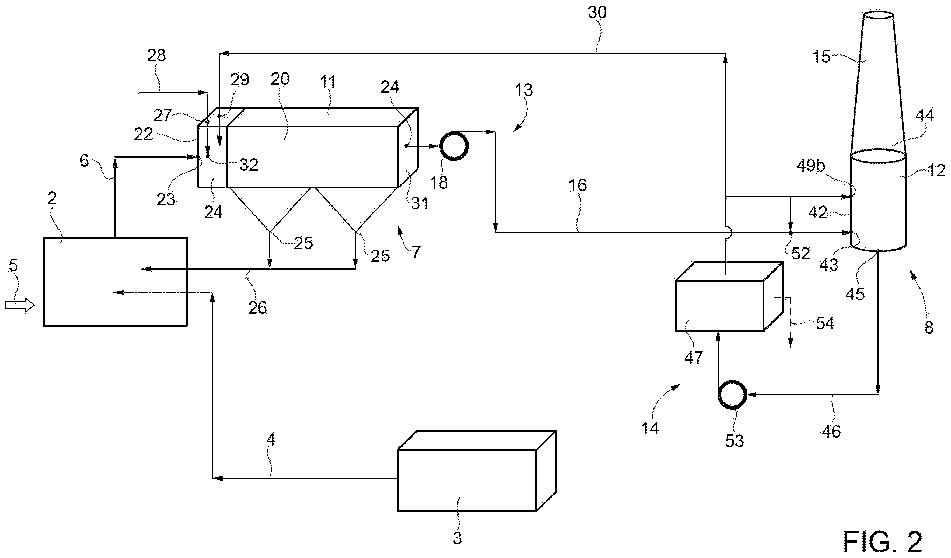

In the embodiment in Figure 2, where details similar or identical to those already described are indicated with the same reference numerals, the second electrostatic precipitator 12 for removing ammonia is a circular-plate electrostatic precipitator and integrates the stack 15.

In this configuration, the electrostatic precipitator 12 has a shell that extends into the stack 15; the gas outlet 44 of the electrostatic precipitator 12 flows directly into the stack 15.

The gas stream to be treated still arrives through the connecting line 16 and enters electrostatic precipitator 12 through gas inlet 43.

Also in this embodiment, the ammonia removal efficiency of the electrostatic precipitator 12 is increased by conditioning with atomized ammonium bisulphate, fed to inlet end 42.

In the example shown in Figure 2, the conditioner (a solution of ammonium bisulphate) is injected into the gas stream via an injector device 52 equipped with an atomizer and located along the connecting line 16.

The solution containing the urea removed from the gas stream and the ammonium sulphate produced in the removal process is extracted from electrostatic precipitator 12 through at least one bottom outlet 45 and sent, through the recovery line 46, to the recovery unit 47 where the ammonium sulphate is degraded (through heating) to form ammonia, which is sent to the urea plant 3, and ammonium bisulphate, which is recirculated to electrostatic precipitator 11 and to electrostatic precipitator 12 through the recirculation line 30. The ammonium bisulphate enters electrostatic precipitator 11 from recirculation inlet 29 and electrostatic precipitator 12 from recirculation inlet 49b; part of the ammonium bisulphate is instead diverted to injector device 52 and flows into the gas line 6 before gas inlet 43.

## Claims

1. A urea plant (3) comprising a solidification unit (2) and a cleaning system (1) for cleaning a gas stream from said solidification unit (2) and containing urea powder and ammonia; the cleaning system (1) comprising a first removal device (7) for removing urea powder and a second removal device (8) for removing ammonia, the two removal devices (7, 8) being arranged in series along a gas treatment circuit (13) and connected by a connecting line (16); **characterized in that** both the two removal devices (7, 8) are electrostatic precipitators (11, 12) designed to remove substantially all or at least most of the contaminant treated in the removal device (7, 8), i.e. urea powder and ammonia; wherein a first electrostatic precipitator (11) constitutes the first removal device (7) for removing urea powder and is a 'dry' electrostatic precipitator, i.e. an electrostatic precipitator operating so as to recover a substantially solid, even if not entirely dry, product from the gas stream; and a second electrostatic precipitator (12) constitutes the second removal device (8) for removing ammonia and is a 'wet' electrostatic precipitator, i.e. an electrostatic precipitator operating so as to recover a liquid or partially liquid product from the gas stream; the cleaning system (1) comprising at least one injector device (52) for injecting a conditioner, in particular ammonium bisulphate, into the gas stream for treatment in the second electrostatic precipitator (12); and wherein the conditioner, e.g. ammonium bisulphate, reacts with ammonia to form a by-product containing the ammonium group, e.g. ammonium sulphate; and the second electrostatic precipitator (12) has one or more bottom outlets (45) connected by a recovery line (46) to a recovery unit (47) equipped with heating means to thermally degrade the by-product produced in the second electrostatic precipitator (12) and form said conditioner and ammonia; the recovery unit (47) being connected by a recirculation line (30) to at least one recirculation inlet (49a, 49b) of the second electrostatic precipitator (12), to feed said conditioner to the second electrostatic precipitator (12).

2. A urea plant according to claim 1, wherein the gas treatment circuit (13) comprises at least one fan (18) located along the connecting line (16) between the removal devices (7, 8) to circulate the gas stream.

3. A urea plant according to claim 1 or 2, wherein the first electrostatic precipitator (11) is a flat-plate 'dry' electrostatic precipitator.

4. A urea plant according to one of the preceding claims, comprising a first injector device (32) for injecting the conditioner into the gas stream for treatment in the first electrostatic precipitator (11); and a second injector device (52) for injecting the conditioner into the gas stream for treatment in the second electrostatic precipitator (12).

5. A urea plant according to claim 4, wherein the first injector device (32) is located in a conditioning region (21) of the first electrostatic precipitator (11) upstream from an electrode region (20) designed for removing urea powder; or upstream from the first electrostatic precipitator (11), e.g. inside the solidification unit (2).

6. A urea plant according to claim 4 or 5, wherein the first injector device (32) is located at a recirculation inlet (29) of the first electrostatic precipitator (11), connected by a recirculation line (30) to said recovery unit (47).

7. A urea plant according to one of claims 4 to 6, wherein the second injector device (52) is located in a conditioning region (41) of the second electrostatic precipitator (12) upstream from an electrode region (40) designed for removing ammonia; or along the connecting line (16) upstream from a gas inlet (43) of the second electrostatic precipitator (12) and upstream from a fan (18) located along the connecting line (16) to circulate the gas stream.

8. A urea plant according to any of claims 1 to 7, wherein the second electrostatic precipitator (12) is a circular- or flat-plate tubular electrostatic precipitator.

9. A urea plant according to any of claims 1 to 7, wherein the second electrostatic precipitator (12) is a circular-plate electrostatic precipitator and incorporates a stack (15); the second electrostatic precipitator (12) having a casing which extends inside the stack (15).

10. A method of cleaning a gas stream from a solidification unit (2) of a urea plant (3) and containing urea powder and ammonia, comprising a urea powder removal step and an ammonia removal step performed in respective removal devices (7, 8) arranged in series along a gas treatment circuit (13) and connected by a connecting line (16); and the cleaning method being **characterized in that** the urea powder removal step is performed entirely or mostly in a first electrostatic precipitator (11), which is a 'dry' electrostatic precipitator, i.e. an electrostatic precipitator operating so as to recover a substantially solid, even if not entirely dry, product from the gas stream; and the ammonia removal step is performed entirely or mostly in a second electrostatic precipitator (12), which is a 'wet' electrostatic precipitator, i.e. an electrostatic precipitator operating so as to recover a liquid or partially liquid product from the gas stream; the cleaning method comprising a conditioning step, in which the gas stream treated in the ammonia removal step is conditioned by adding a conditioner, in particular ammonium bisulphate; and wherein the conditioner, e.g. ammonium bisulphate, reacts with ammonia to form a by-product containing the ammonium group, e.g. ammonium sulphate; and the method comprises the steps of: recovering the by-product from one or more bottom outlets (45) of the second electrostatic precipitator (12); thermally degrading the by-product and forming the conditioner and ammonia; and recirculating the conditioner to the second electrostatic precipitator (12).

11. A cleaning method according to claim 10, comprising a first conditioning step, in which the gas stream treated in the urea powder removal step is conditioned by adding the conditioner; and a second conditioning step, in which the gas stream treated in the ammonia removal step is conditioned by adding the conditioner.

12. A cleaning method according to claim 11, wherein the conditioner is injected into a conditioning region (21) of the first electrostatic precipitator (11) upstream from an electrode region (20) designed for removing urea powder; or upstream from the first electrostatic precipitator (11), e.g. inside the solidification unit (2).

13. A cleaning method according to claim 11 or 12, comprising a step of recirculating to the first electrostatic precipitator (11) the conditioner produced in the recovery unit (47) by thermally degrading the by-product, e.g. ammonium sulphate, produced in the ammonia removal step by the conditioner reacting with ammonia.

14. A cleaning method according to one of claims 10 to 13, wherein the conditioner is injected into a conditioning region (41) of the second electrostatic precipitator (12) upstream from an electrode region (40) designed for removing ammonia; or along the connecting line (16) upstream from a gas inlet (43) of the second electrostatic precipitator (12) and upstream from a fan (18) located along the connecting line (16) to circulate the gas stream.

## Patentansprüche

1. Harnstoffanlage (3), umfassend eine Verfestigungseinheit (2) und ein Reinigungssystem (1) zum Reinigen eines Gasstroms von der Verfestigungseinheit (2), der Harnstoffpulver und Ammoniak enthält; wobei das Reinigungssystem (1) eine erste Entfernvorrichtung (7) zum Entfernen von Harnstoffpulver und eine zweite Entfernvorrichtung (8) zum Entfernen von Ammoniak umfasst, wobei die zwei Entfernvorrichtungen (7, 8) entlang eines Gasaufbereitungskreislaufs (13) hintereinandergeschaltet sind und mittels einer Verbindungsleitung (16) verbunden sind; **dadurch gekennzeichnet, dass** beide der zwei Entfernvorrichtungen (7, 8) Elektroabscheider (11, 12) sind, die darauf ausgerichtet sind, im Wesentlichen alles oder zumindest das meiste der Verunreinigung abzuscheiden, die in der Entfernvorrichtung (7, 8) aufbereitet wird, d. h. Harnstoffpulver und Ammoniak; wobei ein erster Elektroabscheider (11) die erste Entfernvorrichtung (7) zum Entfernen von Harnstoffpulver bildet und ein "trockener" Elektroabscheider ist, d. h. ein Elektroabscheider, der so arbeitet, dass er ein im Wesentlichen festes, wenn auch nicht vollständig trockenes, Produkt von dem Gasstrom rückgewinnt; und wobei ein zweiter Elektroabscheider (12) die zweite Entfernvorrichtung (8) zum Entfernen von Ammoniak bildet und ein "nasser" Elektroabscheider ist, d. h. ein Elektroabscheider, der so arbeitet, dass er ein flüssiges oder teilweise flüssiges Produkt von dem Gasstrom rückgewinnt; wobei das Reinigungssystem (1) mindestens eine Einspritzvorrichtung (52) zum Einspritzen eines Zusatzstoffs, insbesondere Ammoniumhydrogensulfat, in den Gasstrom zur Aufbereitung in dem zweiten Elektroabscheider (12) umfasst; und wobei der Zusatzstoff, z. B. Ammoniumhydrogensulfat, mit Ammoniak reagiert, um ein Nebenprodukt zu bilden, das die Ammoniumgruppe enthält, z. B. Ammoniumsulfat; und wobei der zweite Elektroabscheider (12) einen oder mehrere untere Auslässe (45) aufweist, die mittels einer Rückgewinnungsleitung (46) mit einer Rückgewinnungseinheit (47) verbunden sind, die mit Heizmitteln ausgestattet sind, um das Nebenprodukt, das in dem zweiten Elektroabscheider (12) hergestellt wurde, thermisch abzubauen und den Zusatzstoff und Ammoniak zu bilden; wobei die Rückgewinnungseinheit (47) mittels einer Rücklaufleitung (30) mit mindestens einem Rücklaufeinlass (49a, 49b) des zweiten Elektroabscheiders (12) verbunden ist, um den Zusatzstoff dem zweiten Elektroabscheider (12) zuzuführen.

2. Harnstoffanlage nach Anspruch 1, wobei der Gasaufbereitungskreislauf (13) mindestens einen Ventilator (18) umfasst, der sich entlang der Verbindungsleitung (16) zwischen den Entfernvorrichtungen (7, 8) befindet, um den Gasstrom zu zirkulieren.

3. Harnstoffanlage nach Anspruch 1 oder 2, wobei der erste Elektroabscheider (11) ein "trockener" Flachplattenelektroabscheider ist.

4. Harnstoffanlage nach einem der vorhergehenden Ansprüche, umfassend eine erste Einspritzvorrichtung (32) zum Einspritzen des Zusatzstoffs in den Gasstrom zur Aufbereitung in dem ersten Elektroabscheider (11); und eine zweite Einspritzvorrichtung (52) zum Einspritzen des Zusatzstoffs in den Gasstrom zur Aufbereitung in dem zweiten Elektroabscheider (12).

5. Harnstoffanlage nach Anspruch 4, wobei sich die erste Einspritzvorrichtung (32) in einem Vorbereitungsbereich (21) des ersten Elektroabscheiders (11) befindet, stromaufwärts vor einem Elektrodenbereich (20), der zum Entfernen von Harnstoffpulver ausgestaltet ist; oder stromaufwärts vor dem ersten Elektroabscheider (11), z. B. innerhalb der Verfestigungseinheit (2).

6. Harnstoffanlage nach Anspruch 4 oder 5, wobei sich die erste Einspritzvorrichtung (32) an einem Rückführungseinlass (29) des ersten Elektroabscheiders (11) befindet, die mittels einer Rückführungsleitung (30) mit der Rückgewinnungseinheit (47) verbunden ist.

7. Harnstoffanlage nach einem der Ansprüche 4 bis 6, wobei sich die zweite Einspritzvorrichtung (52) in einem Vorbereitungsbereich (41) des zweiten Elektroabscheiders (12) stromaufwärts vor einem Elektrodenbereich (40) befindet, der für das Entfernen von Ammoniak ausgestaltet ist; oder entlang der Verbindungslinie (16) stromaufwärts vor einem Gaseinlass (43) des zweiten Elektroabscheiders (12) und stromaufwärts vor einem Ventilator (18), der sich entlang der Verbindungslinie (16) befindet, um den Gasstrom zu zirkulieren.

8. Harnstoffanlage nach einem der Ansprüche 1 bis 7, wobei der zweite Elektroabscheider (12) ein Kreisplatten- oder Flachplatten-Röhrenelektroabscheider ist.

9. Harnstoffanlage nach einem der Ansprüche 1 bis 7, wobei der zweite Elektroabscheider (12) ein Kreisplatten-Elektroabscheider ist und einen Schornstein (15) verkörpert; wobei der zweite Elektroabscheider (12) eine Ummantelung aufweist, die sich in das Innere des Schornsteins (15) erstreckt.

10. Verfahren zum Reinigen eines Gasstroms von einer Verfestigungseinheit (2) einer Harnstoffanlage (3), der Harnstoffpulver und Ammoniak enthält, umfassend einen Harnstoffpulver-Entfernschritt und einen Ammoniak-Entfernschritt, die in jeweiligen Entfernvorrichtungen (7, 8) durchgeführt werden, die entlang eines Gasaufbereitungskreislaufs (13) hintereinandergeschaltet sind und mittels einer Verbindungsleitung (16) verbunden sind; und wobei das Reinigungsverfahren **dadurch gekennzeichnet ist, dass** der Harnstoffpulver-Entfernschritt vollständig oder größtenteils in einem ersten Elektroabscheider (11) durchgeführt wird, der ein "trockener" Elektroabscheider ist, d. h. ein Elektroabscheider, der so arbeitet, dass er ein im Wesentlichen festes, wenn auch nicht vollständig trockenes, Produkt von dem Gasstrom rückgewinnt; und wobei der Ammoniak-Entfernschritt vollständig oder größtenteils in einem zweiten Elektroabscheider (12) durchgeführt wird, der ein "nasser" Elektroabscheider ist, d. h. ein Elektroabscheider, der so arbeitet, dass er ein flüssiges oder teilweise flüssiges Produkt von dem Gasstrom rückgewinnt; wobei das Reinigungsverfahren einen Vorbereitungsschritt umfasst, bei dem der Gasstrom, der in dem Ammoniak-Entfernschritt aufbereitet wird, durch Hinzufügen eines Zusatzstoffs, insbesondere Ammoniumhydrogensulfat, vorbereitet wird; und wobei der Zusatzstoff, z. B. Ammoniumhydrogensulfat, mit Ammoniak reagiert, um ein Nebenprodukt zu bilden, das die Ammoniumgruppe enthält, z. B. Ammoniumsulfat; und das Verfahren folgende Schritte umfasst: Rückgewinnen des Nebenprodukts von einem oder mehreren unteren Auslässen (45) des zweiten Elektroabscheiders (12); thermisches Abbauen des Nebenprodukts und Bilden des Zusatzstoffs und Ammoniaks; und Rückführen des Zusatzstoffs zu dem zweiten Elektroabscheider (12).

11. Reinigungsverfahren nach Anspruch 10, umfassend einen ersten Vorbereitungsschritt, in dem der Gasstrom, der in dem Harnstoffpulver-Entfernschritt aufbereitet wird, durch Hinzufügen des Zusatzstoffs vorbereitet wird; und einen zweiten Vorbereitungsschritt, in dem der Gasstrom, der in dem Ammoniak-Entfernschritt aufbereitet wird, durch Hinzufügen des Zusatzstoffs vorbereitet wird.

12. Reinigungsverfahren nach Anspruch 11, wobei der Zusatzstoff in einen Vorbereitungsbereich (21) des ersten Elektroabscheiders (11) stromaufwärts vor einem Elektrodenbereich (20), der für das Entfernen von Harnstoffpulver ausgestaltet ist, eingespritzt wird; oder stromaufwärts vor dem ersten Elektroabscheider (11), z. B. innerhalb der Verfestigungseinheit (2).

13. Reinigungsverfahren nach Anspruch 11 oder 12, umfassend einen Schritt des Rückführens zu dem ersten Elektroabscheider (11) des Zusatzstoffs, der in der in der Rückgewinnungseinheit (47) hergestellt wird, durch thermisches Abbauen des Nebenprodukts, z. B. Ammoniumsulfat, das in dem Ammoniak-Entfernschritt durch den Zusatzstoff, der mit Ammoniak reagiert, hergestellt wird.

14. Reinigungsverfahren nach einem der Ansprüche 10 bis 13, wobei der Zusatzstoff in einen Vorbereitungsbereich (41) des zweiten Elektroabscheiders (12) stromaufwärts vor einen Elektrodenbereich (40) eingespritzt wird, der zum Entfernen von Ammoniak ausgestaltet ist; oder entlang der Verbindungsleitung (16) stromaufwärts vor einem Gaseinlass (43) des zweiten Elektroabscheiders (12) und stromaufwärts vor einem Ventilator (18), der sich entlang der Verbindungsleitung (16) befindet, um den Gasstrom zirkulieren zu lassen.

## Revendications

1. Installation de production d'urée (3) comprenant une unité de solidification (2) et un système d'épuration (1) pour épurer un courant gazeux provenant de ladite unité de solidification (2) et contenant une poudre d'urée et de l'ammoniac ; le système d'épuration (1) comprenant un premier dispositif d'élimination (7) pour éliminer une poudre d'urée et un second dispositif d'élimination (8) pour éliminer l'ammoniac, les deux dispositifs d'élimination (7, 8) étant agencés en série le long d'un circuit de traitement de gaz (13) et raccordés par une conduite de raccordement (16) ; **caractérisée en ce qu'**à la fois les deux dispositifs d'élimination (7, 8) sont des précipitateurs électrostatiques (11, 12) conçus pour éliminer sensiblement la totalité ou au moins une majeure partie du contaminant traité dans le dispositif d'élimination (7, 8), c'est-à-dire une poudre d'urée et de l'ammoniac ; dans laquelle un premier précipitateur électrostatique (11) constitue le premier dispositif d'élimination (7) pour éliminer une poudre d'urée et est un précipitateur électrostatique « sec », c'est-à-dire un précipitateur électrostatique fonctionnant afin de récupérer un produit sensiblement solide, même s'il n'est pas entièrement sec, à partir du courant gazeux ; et un second précipitateur électrostatique (12) constitue le second dispositif d'élimination (8) pour éliminer l'ammoniac et est un précipitateur électrostatique « humide », c'est-à-dire un précipitateur électrostatique fonctionnant afin de récupérer un produit liquide ou partiellement liquide à partir du courant gazeux ; le système d'épuration (1) comprenant au moins un dispositif injecteur (52) pour injecter un conditionneur, en particulier du bisulfate d'ammonium, dans le courant gazeux à des fins de traitement dans le second précipitateur électrostatique (12) ; et dans laquelle le conditionneur, par exemple du bisulfate d'ammonium, réagit avec l'ammoniac pour former un sous-produit contenant le groupe ammonium, par exemple du sulfate d'ammonium ; et le second précipitateur électrostatique (12) comporte une ou plusieurs sorties inférieures (45) raccordées par une conduite de récupération (46) à une unité de récupération (47) équipée d'un moyen de chauffage pour dégrader thermiquement le sous-produit produit dans le second précipitateur électrostatique (12) et former ledit conditionneur et l'ammoniac ; l'unité de récupération (47) étant raccordée par une conduite de remise en circulation (30) à au moins une entrée de remise en circulation (49a, 49b) du second précipitateur électrostatique (12), pour introduire ledit conditionneur dans le second précipitateur électrostatique (12).

2. Installation de production d'urée selon la revendication 1, dans laquelle le circuit de traitement de gaz (13) comprend au moins un ventilateur (18) situé le long de la conduite de raccordement (16) entre les dispositifs d'élimination (7, 8) pour faire circuler le courant gazeux.

3. Installation de production d'urée selon la revendication 1 ou 2, dans laquelle le premier précipitateur électrostatique (11) est un précipitateur électrostatique « sec » à plaque plate.

4. Installation de production d'urée selon l'une des revendications précédentes, comprenant un premier dispositif injecteur (32) pour injecter le conditionneur dans le courant gazeux à des fins de traitement dans le premier précipitateur électrostatique (11) ; et un second dispositif injecteur (52) pour injecter le conditionneur dans le courant gazeux à des fins de traitement dans le second précipitateur électrostatique (12).

5. Installation de production d'urée selon la revendication 4, dans laquelle le premier dispositif injecteur (32) est situé dans une région de conditionnement (21) du premier précipitateur électrostatique (11) en amont d'une région d'électrode (20) conçue pour éliminer une poudre d'urée ; ou en amont du premier précipitateur électrostatique (11), par exemple à l'intérieur de l'unité de solidification (2).

6. Installation de production d'urée selon la revendication 4 ou 5, dans laquelle le premier dispositif injecteur (32) est situé au niveau d'une entrée de remise en circulation (29) du premier précipitateur électrostatique (11), raccordé par une conduite de remise en circulation (30) à ladite unité de récupération (47).

7. Installation de production d'urée selon l'une des revendications 4 à 6, dans laquelle le second dispositif injecteur (52) est situé dans une région de conditionnement (41) du second précipitateur électrostatique (12) en amont d'une région d'électrode (40) conçue pour éliminer l'ammoniac ; ou le long de la conduite de raccordement (16) en amont d'une entrée de gaz (43) du second précipitateur électrostatique (12) et en amont d'un ventilateur (18) situé le long de la conduite de raccordement (16) pour faire circuler le courant gazeux.

8. Installation de production d'urée selon l'une quelconque des revendications 1 à 7, dans laquelle le second précipitateur électrostatique (12) est un précipitateur électrostatique tubulaire à plaque circulaire ou plate.

9. Installation de production d'urée selon l'une quelconque des revendications 1 à 7, dans laquelle le second précipitateur électrostatique (12) est un précipitateur électrostatique à plaque circulaire et incorpore une cheminée (15) ; le second précipitateur électrostatique (12) ayant une enveloppe qui se prolonge à l'intérieur de la cheminée (15).

10. Procédé d'épuration d'un courant gazeux provenant d'une unité de solidification (2) d'une installation de production d'urée (3) et contenant une poudre d'urée et de l'ammoniac, comprenant une étape d'élimination de poudre d'urée et une étape d'élimination d'ammoniac effectuées dans des dispositifs d'élimination (7, 8) respectifs agencés en série le long d'un circuit de traitement de gaz (13) et raccordés par une conduite de raccordement (16) ; et le procédé d'épuration étant **caractérisé en ce que** l'étape d'élimination de poudre d'urée est effectuée entièrement ou principalement dans un premier précipitateur électrostatique (11), qui est un précipitateur électrostatique « sec », c'est-à-dire un précipitateur électrostatique fonctionnant afin de récupérer un produit sensiblement solide, même s'il n'est pas entièrement sec, à partir du courant gazeux ; et l'étape d'élimination d'ammoniac est effectuée entièrement ou principalement dans un second précipitateur électrostatique (12), qui est un précipitateur électrostatique « humide », c'est-à-dire un précipitateur électrostatique fonctionnant afin de récupérer un produit liquide ou partiellement liquide à partir du courant gazeux ; le procédé d'épuration comprenant une étape de conditionnement, dans laquelle le courant gazeux traité dans l'étape d'élimination d'ammoniac est conditionné par l'ajout d'un conditionneur, en particulier du bisulfate d'ammonium ; et dans laquelle le conditionneur, par exemple du bisulfate d'ammonium, réagit avec l'ammoniac pour former un sous-produit contenant le groupe ammonium, par exemple du sulfate d'ammonium ; et le procédé comprend les étapes suivantes : la récupération du sous-produit à partir d'une ou de plusieurs sorties inférieures (45) du second précipitateur électrostatique (12) ; la dégradation thermique du sous-produit et la formation du conditionneur et de l'ammoniac ; et la remise en circulation du conditionneur vers le second précipitateur électrostatique (12).

11. Procédé d'épuration selon la revendication 10, comprenant une première étape de conditionnement, dans laquelle le courant gazeux traité dans l'étape d'élimination de poudre d'urée est conditionné par l'ajout du conditionneur ; et une seconde étape de conditionnement, dans lequel le courant gazeux traité dans l'étape d'élimination d'ammoniac est conditionné par l'ajout du conditionneur.

12. Procédé d'épuration selon la revendication 11, dans lequel le conditionneur est injecté dans une région de conditionnement (21) du premier précipitateur électrostatique (11) en amont d'une région d'électrode (20) conçue pour éliminer une poudre d'urée ; ou en amont du premier précipitateur électrostatique (11), par exemple à l'intérieur de l'unité de solidification (2).

13. Procédé d'épuration selon la revendication 11 ou 12, comprenant une étape de remise en circulation vers le premier précipitateur électrostatique (11) du conditionneur produit dans l'unité de récupération (47) par la dégradation thermique du sous-produit, par exemple du sulfate d'ammonium, produit dans l'étape d'élimination d'ammoniac par la réaction du conditionneur avec l'ammoniac.

14. Procédé d'épuration selon l'une des revendications 10 à 13, dans lequel le conditionneur est injecté dans une région de conditionnement (41) du second précipitateur électrostatique (12) en amont d'une région d'électrode (40) conçue pour éliminer l'ammoniac ; ou le long de la conduite de raccordement (16) en amont d'une entrée de gaz (43) du second précipitateur électrostatique (12) et en amont d'un ventilateur (18) situé le long de la conduite de raccordement (16) pour faire circuler le courant gazeux.
